(19) Europäisches Patentamt
European Patent Office
Office européen des brevets

(11) **EP 2 157 905 B1**

(12) **EUROPEAN PATENT SPECIFICATION**

(45) Date of publication and mention of the grant of the patent:
**27.03.2013 Bulletin 2013/13**

(21) Application number: **08757093.3**

(22) Date of filing: **15.05.2008**

(51) Int Cl.:
*A61B 5/00* *(2006.01)*    *A61B 6/03* *(2006.01)*
*G06T 15/00* *(2011.01)*    *G06T 7/00* *(2006.01)*
*G06T 7/60* *(2006.01)*

(86) International application number:
**PCT/CA2008/000933**

(87) International publication number:
**WO 2008/138140 (20.11.2008 Gazette 2008/47)**

(54) **A METHOD FOR TRACKING 3D ANATOMICAL AND PATHOLOGICAL CHANGES IN TUBULAR-SHAPED ANATOMICAL STRUCTURES**

VERFAHREN ZUR VERFOLGUNG VON DREIDIMENSIONALEN ANATOMISCHEN UND PATHOLOGISCHEN VERÄNDERUNGEN IN RÖHRENFÖRMIGEN ANATOMISCHEN STRUKTUREN

PROCÉDÉ DE SUIVI DES CHANGEMENTS ANATOMIQUES ET PATHOLOGIQUES EN 3 DIMENSIONS DANS DES STRUCTURES ANATOMIQUES DE FORME TUBULAIRE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.05.2007 US 938078 P**

(43) Date of publication of application:
**03.03.2010 Bulletin 2010/09**

(73) Proprietors:
• **Ecole de Technologie Supérieure**
  **Montréal,**
  **Québec H3C 1K3 (CA)**
• **Val-Chum, Limited Partnership**
  **Montréal, QC H3V 1H8 (CA)**

(72) Inventors:
• **KAUFFMANN, Claude**
  **Montreal, Quebec H2L 4M1 (CA)**

• **SOULEZ, Gilles**
  **Outremont**
  **Québec H2V 2Z2 (CA)**
• **DE GUISE, Jacques A.**
  **Montréal**
  **Québec H2W 2L7 (CA)**

(74) Representative: **Kuhnen & Wacker**
  **Patent- und Rechtsanwaltsbüro**
  **Prinz-Ludwig-Strasse 40A**
  **85354 Freising (DE)**

(56) References cited:
WO-A1-2006/085155   WO-A2-03/100542
WO-A2-2005/069223   CA-A1- 2 444 364
US-A1- 2002 118 869   US-A1- 2005 085 709

**Description**

FIELD OF THE INVENTION

The present invention relates to a method for tracking 3D anatomical and pathological changes in tubular-shaped anatomical structures.

BACKGROUND OF THE INVENTION

Medical imaging is increasingly used to study the changes in size and shape of anatomical structures over time. As these changes often serve as indicators of the presence of a disease, extraction of quantitative information from such medical images has many applications in clinical diagnosis.

Conventional practice is to outline anatomical structures by image segmentation, a fundamental step of image analysis, during which anatomical and pathological structure information is typically extracted from patient image data. Image segmentation allows various relevant anatomical structures to be distinguished, which often have similar intensity values on the image and thus overlap or are interrelated. Performing the segmentation directly in the three-dimensional (3D) space brings more consistency in the results. The method enables clinicians to emphasize and extract various features in the digital images by partitioning them into multiple regions, thereby delimiting image areas representing objects of interest, such as organs, bones, and different tissue types. Although different segmentation approaches have been applied in different situations, the common principle lies in the iterative process, which progressively improves the resulting segmentation so that it gradually corresponds better to a certain a priori image interpretation. Still, currently practiced methods take a significant amount of time to extract information from the medical images, and as a result do not achieve optimal results in a fast and efficient manner.

Medical imaging has proven particularly effective in the diagnosis of pathologies such as aortic aneurysms, a fairly common disorder characterized by a localized dilation greater than 1.5 times the typical diameter of the aorta. As rupture of the aneurysm, which is the main complication of the disorder, typically results in death due to internal bleeding, accurate diagnosis and control of the aneurysm are critical. The main predictors of rupture risk are the maximal diameter (Dmax) and the expansion rate of the aneurysm. It has been suggested that a Dmax value greater than 5.5 cm in men and 4.5 to 5.0 cm in women, as well as an expansion rate greater than 1 cm per year are indications for a procedure. Study of these parameters is therefore crucial in determining when a surgical intervention is warranted to prevent the aneurysm from rupturing or causing other complications in the future.

The prior art teaches various methods for computing the value of Dmax, leading to different inconsistent definitions of the Dmax parameter. In addition, current measurement methods typically generate intra- and inter-observer variability as well as result in systematic overestimation of the Dmax value as they use either rough estimation based on the appearance of the aneurysm or cumbersome and time-consuming manual outlining of aneurysm anatomy or pathology on sequences of patient images. Also, as current segmentation techniques use contrast agents that only enable visualization of the aneurysm lumen and not visualization of the thrombus, the latter cannot be segmented using these methods, although it is critical in determining the value of Dmax. Current segmentation techniques further make it difficult to control the quality of the segmentation as well as correct any mistakes generated by the software.

Additionally, WO 2006/085155 discloses a method for imaging a volume of interest, such as an organ or tumour or the like, where the data slices contain a contour line which when stacked together define the volume and from which a plurality of curves are calculated. The curves are arranged to bound the volume.

What is therefore needed, and an object of the present invention, is a standardized method for tracking 3D changes in an anatomical structure, such as an aortic aneurysm, based on 3D images. In particular, a clinical diagnostic tool, which enables segmentation of medical images in 3D to be performed and accurate information related to the anatomical structure under observation obtained in a simple, fast and reproducible manner, would be useful.

SUMMARY OF THE INVENTION

In order to address the above and other drawbacks, there is disclosed a method for visualizing an anatomy of a region of interest of a tubular-shaped organ on a display. The method comprises acquiring an image of the anatomy of the tubular shaped organ in the region of interest at a first point in time, extracting a plurality of discrete points from the image defining a minimum-curvature path within the tubular-shaped organ, interpolating a set of cross-sectional images along planes substantially perpendicular to a tangent vector of the minimum-curvature path at each of the plurality of discrete points, delimiting a segmented area corresponding to the region of interest of the tubular-shaped organ in each of the set of cross-sectional images, rendering a three-dimensional surface representation of the region of interest from the delimited set of cross-sectional images and displaying the rendered three-dimensional surface representation on the display.

Additionally, there is disclosed a system for visualizing the anatomy of a region of interest of a tubular-shaped organ. The system comprises a scanning device for acquiring an image of the region of interest of the tubular shaped organ, a database connected to the scanning device for storing the acquired image, and a workstation connected to the database for retrieving the stored image, the workstation comprising a display, a user interface, and an image processor. Responsive to the commands from the user interface, the image processor extracts from the image a plurality of discrete points defining a minimum-curvature path within the region of interest of the tubular-shaped organ, interpolates a set of cross-sectional images along planes substantially perpendicular to a tangent vector of the minimum-curvature path at each of the plurality of discrete points, delimits a segmented area corresponding to the region of interest of the tubular-shaped organ in each of the set of cross-sectional images, computes a three-dimensional surface representation of the region of interest from the delimited set of cross-sectional images and displays the computed three-dimensional surface representation on the display.

Furthermore, there is disclosed a computer program storage medium readable by a computing system and encoding a computer program of instructions for executing a computer process for visualizing the anatomy of a region of interest of a tubular-shaped organ. The computer process comprises acquiring an image of the anatomy of the tubular shaped organ in the region of interest, extracting from the image a plurality of discrete points defining a minimum-curvature path within the tubular-shaped organ, interpolating a set of cross-sectional images along planes substantially perpendicular to a tangent vector of the minimum-curvature path at each of the discrete points, delimiting a segmented area corresponding to the region of interest of the tubular-shaped organ in each of the set of cross-sectional images, computing a three-dimensional surface representation of the region of interest from the delimited set of cross-sectional images, and displaying the rendered three-dimensional surface representation on the display.

Other objects, advantages and features of the present invention will become more apparent upon reading of the following non-restrictive description of specific embodiments thereof, given by way of example only with reference to the accompanying drawings.

BRIEF DESCRIPTION OF THE DRAWINGS

In the appended drawings:

Figure 1 is a schematic diagram of an image analysis system in accordance with an illustrative embodiment of the present invention;

Figure 2 is a flow chart of an image analysis method in accordance with an illustrative embodiment of the present invention;

Figure 3 is a diagram of an abdominal aortic aneurysm in accordance with an illustrative embodiment of the present invention;

Figures 4a and 4b show cross-section images of the abdominal aortic aneurysm of Figure 3 during landmark initialization in accordance with an illustrative embodiment of the present invention;

Figure 5 shows a cross-section image of an abdominal aortic aneurysm interpolated along a minimum-curvature path in accordance with an illustrative embodiment of the present invention;

Figures 6a and 6b show a representation of cross-section images used for segmentation of an abdominal aortic aneurysm in accordance with an illustrative embodiment of the present invention;

Figures 7a and 7b show the cross-section images of Figures 6a and 6b during positioning of angular slices in accordance with an illustrative embodiment of the present invention;

Figures 8a and 8b show cross-section images of an abdominal aortic aneurysm during active-shape contour segmentation in accordance with an illustrative embodiment of the present invention;

Figures 9a and 9b show cross-section images of an abdominal aortic aneurysm during segmentation quality control in accordance with an illustrative embodiment of the present invention;

Figure 10 is a schematic diagram of a 3D aneurysm wall model in accordance with an illustrative embodiment of the present invention;

Figure 11 is a representation of the 3D aneurysm wall model of Figure 10 in axial, sagittal and coronal views in accordance with an illustrative embodiment of the present invention;

Figures 12a and 12b show two representations of the maximum diameter of an abdominal aortic aneurysm in accordance with an illustrative embodiment of the present invention;

Figure 13a shows a segmentation of the false thrombus an aorta in accordance with an illustrative embodiment of the present invention;

Figure 13b shows a segmentation of an aorta separated into two pathological components resulting from aortic dissection in accordance with an illustrative embodiment of the present invention;

Figure 14a shows a segmentation of the lumen of a thoracic aortic aneurysm in accordance with an illustrative embodiment of the present invention;

Figures 14b and 14c show a segmentation of the thrombus and a representation on a 3D wall model of the maximum diameter of a thoracic aortic aneurysm in accordance with an illustrative embodiment of the present invention;

Figure 14d and 14e show a representation on a 3D wall model of the thrombus thickness of a thoracic aortic aneurysm in accordance with an illustrative embodiment of the present invention;

Figure 15 shows a segmentation of a cat's spinal cord in accordance with an illustrative embodiment of the present invention;

Figure 16 is a flow chart of an image registration method in accordance with an illustrative embodiment of the present invention; and

Figure 17 is a schematic of an abdominal aortic aneurysm during landmark initialization for image registration in accordance with an illustrative embodiment of the present invention.

DETAILED DESCRIPTION OF THE ILLUSTRATIVE EMBODIMENTS

The present invention is illustrated in further details by the following nonlimiting examples.

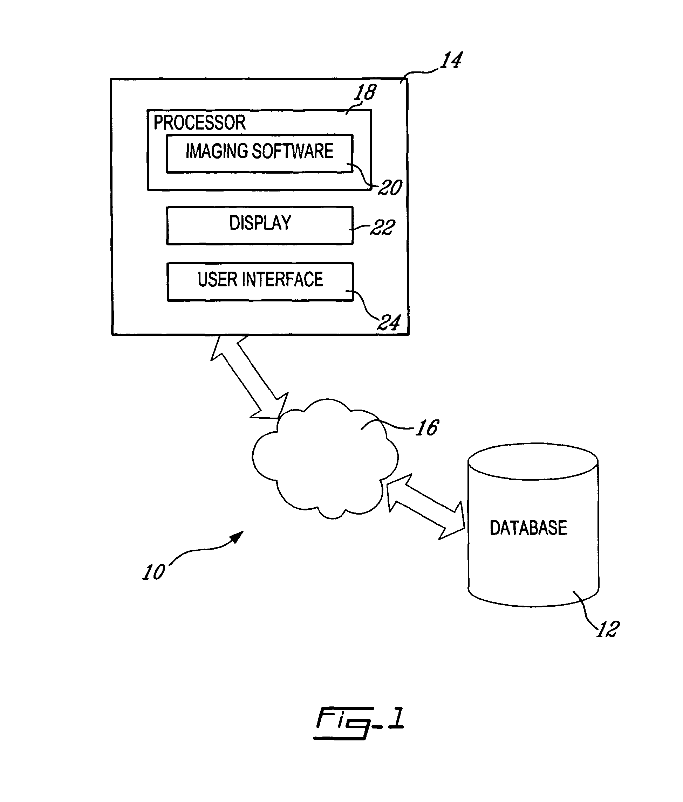

Referring to Figure 1, and in accordance with an illustrative embodiment of the present invention, a system for processing and analyzing medical images, generally referred to using the reference numeral 10, will now be described. The system 10 comprises a database 12 for storing patient images and a workstation 14 for accessing the stored images through a communications network 16, such as a Local Area Network (LAN). The workstation 14 comprises a processor 18, on which an imaging software module 20 responsible for processing images retrieved from the database 12 is installed. The workstation 14 further comprises a display 22 and a user interface 24 (e.g. a mouse and keyboard), which enable users to interact with the imaging software 20 by displaying and manipulating image data in response to input commands. The display 22 and the user interface 24 thus enable users to visualize and supervise the image analysis process performed by the imaging software 20.

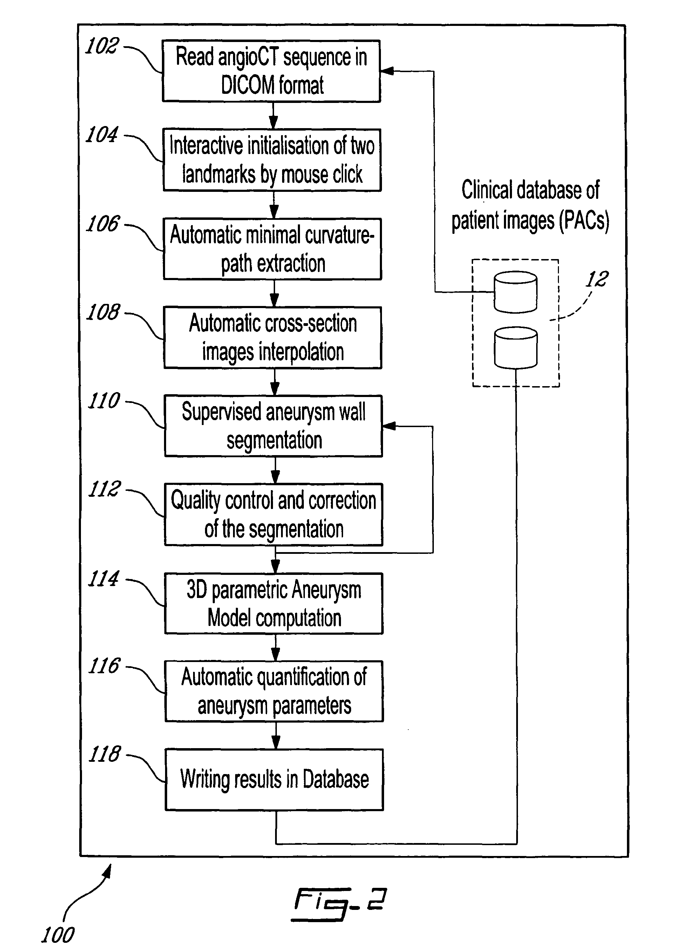

Referring now to Figure 2 in addition to Figure 1, a medical image analysis method 100 implemented by the imaging software 20 will now be described. Clinical image data related to a patient under observation is typically acquired by a scanner (not shown) of a standard medical imaging modality such as Computed Tomography (CT) or Magnetic Resonance Imaging (MRI) angiography. Angiography has the advantage of being an efficient and relatively non-invasive diagnostic tool. Illustratively, in CT angiography, an X-ray picture is taken to visualize the inner opening of blood filled structures, including arteries, veins and the heart chambers. Contrast agents may be used to improve the visibility of the patient's internal bodily structures on the angiography image, for instance by enabling to differentiate intensity values of the vessel interior and wall. Thin axial image slices of the area under observation are typically obtained during the procedure and images in the remaining two spatial planes (coronal and sagittal) are calculated by a computer. After their acquisition, the patient images are stored as image data sets into the database 12, illustratively in the Digital Image Communications in Medicine (DICOM) format, for subsequent retrieval and analysis. DICOM format is of particular interest in medical applications, as it enables easy standardised data communication between systems produced by different manufacturers and using different internal formats, thus allowing effective connection of different components of an imaging department. Since different clinical imaging exams may be performed at different times to study the progression of a patient's disorder, a resulting plurality of image data sets corresponding to each imaging exam may be stored in the database 12 and each image set is then treated separately by the imaging software 20.

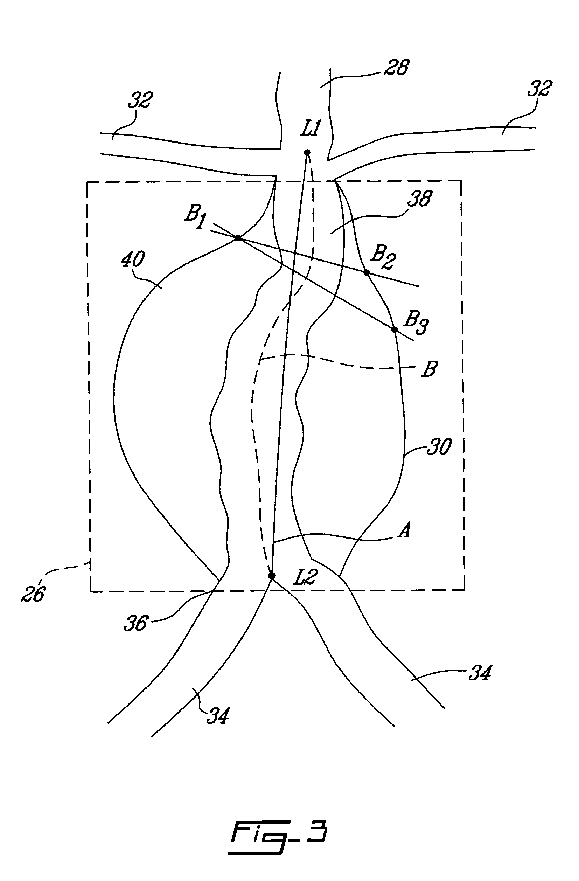

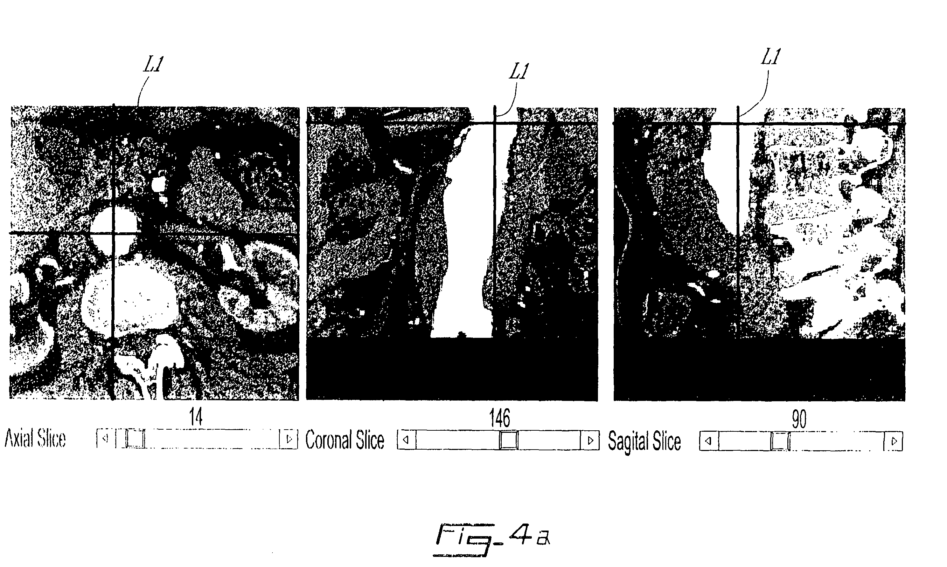

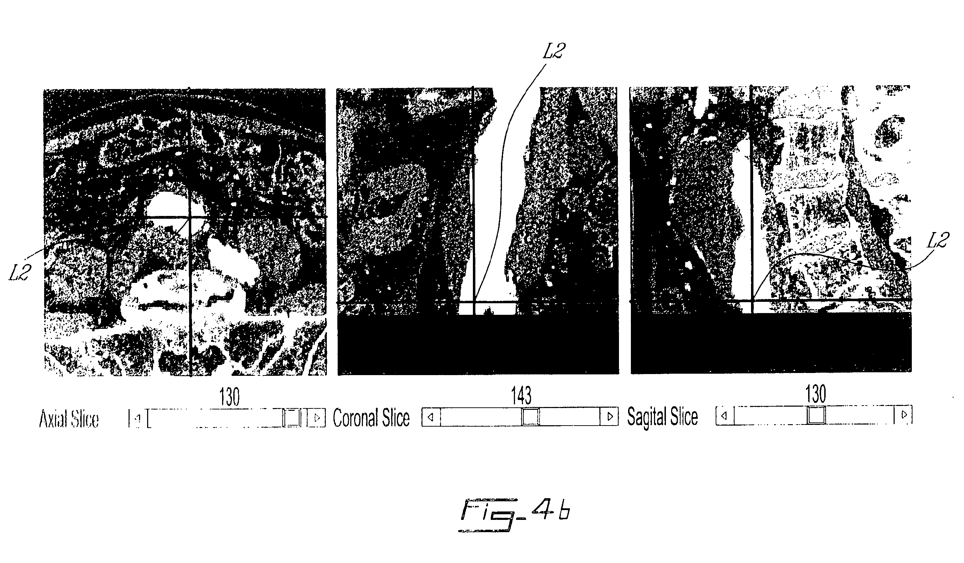

Referring now to Figure 3 and Figures 4a and 4b in addition to Figures 1 and 2, a user wishing to analyze patient images illustratively accesses the workstation 14, and via the user interface 24 (which illustratively comprises, in addition to the display 22, a pointing device such as a mouse or the like and an appropriate operating system software), imports the image set(s) related to the patient under observation. The imaging software 20 is then invoked by the user in order to open the imported images (102), which are shown on the display 22 so that the user may proceed with the segmentation process at 104. For sake of illustration, the anatomical structure under observation is an abdominal aortic aneurysm 26, although it would be understood by one skilled in the art that the method 100 may be applied to other types of aneurysms (e.g. thoracic, intracranial), as well as other tubular-shaped organs, such as the colon, trachea, and spine. The method 100 may also have other applications such as analysis of soft tissues, of atheromatous plaque in carotid arteries, and follow-up of stent grafts.

As illustrated in Figure 3, an abdominal aortic aneurysm 26 is a disorder of the aorta 28 characterized by a localized dilation of the arterial wall 30. An aortic aneurysm is typically located below the renal arteries 32 and above the iliac arteries 34 and the aorta-iliac bifurcation 36. The inner space of the aorta is referred to as the lumen 38, as is the case for any other vessel in the body, while the thickness of the aorta wall in the region of the aneurysm is referred to as the thrombus 40.

Still referring to Figure 3, Figure 4a and Figure 4b in addition to Figure 2, to visualize the aneurysm 26 and initiate the segmentation process of the aneurysm wall 30, the user illustratively defines two displaced landmarks L1 and L2 in characteristic and easily identifiable regions of the lumen 38 and towards either ends of the portion of the lumen 38 to be visualised. This is done via the user interface 24 by moving a cursor in one or other of the displayed axial, coronal and sagittal image slices, as illustrated in Figure 3. Illustratively, a first landmark L1 is placed before the aneurysm 26 (Figure 4a) and a second landmark L2 after the aneurysm 26 (Figure 4b). The user then validates the positions of the landmarks, for example by simple mouse click. Landmark initialization is illustratively done in Multi-Planar Reformatting (MPR) view, a reformatting technique which passes a plane through an image set, thus enabling users to view the volume under inspection along a different direction than that of the original image set. In effect, one can view the image data from different viewpoints without having to rescan the patient.

Still referring to Figure 3, Figure 4a and Figure 4b in addition to Figure 2, the landmarks L1 and L2 thus defined are used at 106 as start and end points for automatic extraction of a minimum-curvature path A (not necessarily straight). It is desirable for the path A, which links landmarks L1 and L2 and has minimal curvature, to be fully defined inside the aneurysm lumen 38. The path A is used to define new cross-section images, which ensure that slicing of the aneurysm 26, leads to proper segmentation of the aneurysm wall 30 and to accurate rendering in 3D. Indeed, as seen on Figure 3, if cross-section images were to be defined along the geometric centreline B of the aneurysm lumen 38 for example, two successive cross-section images taken in areas where the lumen 38 is more irregular might intersect at point B1 on one side of the aneurysm outer wall 30. On the opposite side, each cross-section image would intersect the outer wall 30 at points B2 and B3 but the spacing between these points would be large, leading to a loss in precision, as no additional points would have been obtained to more accurately define the region of the outer wall 30 between B2 and B3. Taking cross-section images along the minimum-curvature path A therefore ensures that none of the cross-section images intersect, resulting in a more precise definition of the contour of the aneurysm 26. Illustratively, the minimum-curvature path A is computed by initially extracting a shortest path between the two landmarks L1 and L2. This shortest path is illustratively obtained using Dijkstra's algorithm, an algorithm which solves shortest-path problems for directed graphs. A matrix of discrete point coordinates $D_p$, which correspond to the lowest-cost (i.e. shortest) path between the two landmarks L1 and L2, is then obtained in the Dijkstra metric. The gray-level values Idp (i.e. the brightness) of each discrete point $D_p$ are further extracted as Idp = Image ($D_p$), using the 3D image (Image) reconstructed from the acquired slices. These values are then used to compute a Fuzzy representation FuzzyImage of the native (i.e. original) images based on a Gaussian distribution centred at the mean value of the gray-level values Idp as follows:

$$\text{FuzzyImage} = \exp(-((\text{Image} - \text{mIdp}).\^2)/(k*(\text{StdIdp})\^2)) \tag{1}$$

with: Image = normalized 3D image
mIdp = mean value of Idp
StdIdp = standard deviation of Idp
k = an integer that controls the width of the Gaussian distribution

Once the Fuzzy images have been computed, a distance-map is illustratively obtained using the fast-Marching algorithm based on the propagation of a wave front starting at landmark point L1. The front propagation is stopped when it reaches landmark point L2 and a distance map, which supplies each point in the image with the distance to the nearest obstacle point (i.e. boundary), is obtained. From this distance map, the minimum-curvature path A between L2 and L1 is computed, illustratively by back propagation from L2 to L1 using an optimization algorithm such as the gradient descent algorithm, in which a local minimum of a function is found by determining successive descent directions and steps from a starting point on the function.

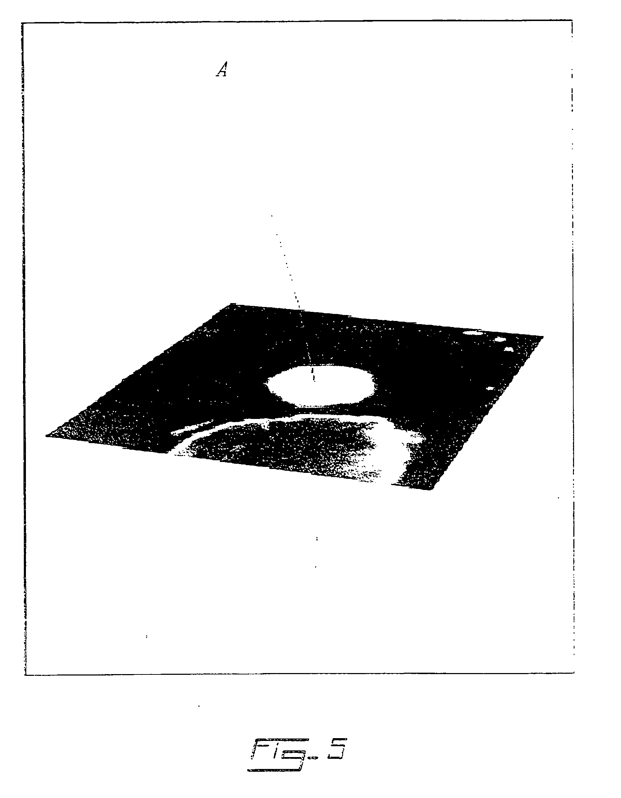

Referring now to Figure 5 in addition to Figure 4a, Figure 4b and Figure 2, at 108, the minimum-curvature path A is then used to interpolate image slices defined by successive cross-sections along the path A. This will result in a new image space of interpolated cross-section images, on which segmentation of the aneurysm will subsequently be performed. For this purpose, a Frenet reference frame is illustratively defined on the path start point (L1 or L2). A Frenet reference frame is a local coordinate system, which can be calculated anywhere along a curve independently from the curve's parameterization and consists of the tangent vector to the curve, the normal vector that points to the centre of the curve and the binormal vector, which is a cross product of the tangent and normal vectors. For each successive discrete point on the path A, the Frenet reference frame is recomputed and the changes in translation and rotation between the actual and precedent frame are evaluated. The precedent frame is then propagated to the actual position using small local rotations in order to obtain a torsion-free frame. Figure 5 shows an example of a cross-section image interpolated at a specific position on the path A. The interpolated cross-section images may be spaced along the path A either regularly or with a spacing function defined by the path's curvature. If a spacing function is used, more cross-sections are computed in the path sections having a high curvature, in order to better define the aneurysm, thus leading to more accurate segmentation.

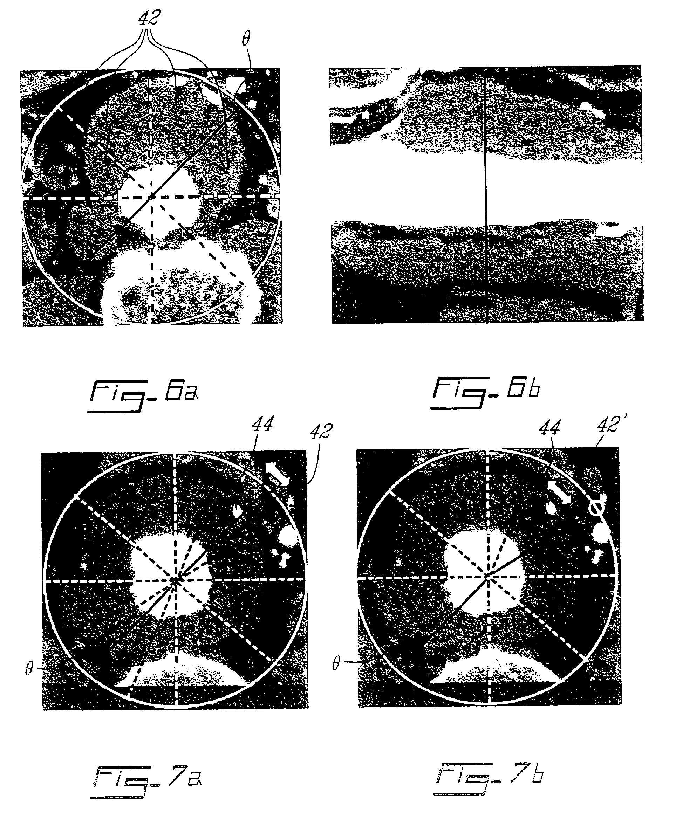

Referring now to Figure 6a, Figure 6b, Figure 7a and Figure 7b in addition to Figure 1, Figure 2 and Figure 3, using the new image space interpolation, two representations of the cross-section images are illustratively used at 110 to segment the aneurysm wall 30: an axial representation (Figure 6a) and an image interpolation along the minimum-curvature path A at a specific angular position θ around it (Figure 6b). Defining angular slices 42 at an angular position θ allows the user to segment the aneurysm wall 30 at a variety of angles θ. Proper selection of the number of slices 42 ensures that the slices 42 pass through certainty areas, i.e. areas of the aneurysm 26 where image information is known, and avoid risk areas (e.g. noise and artifacts) during the segmentation process. The number of angular slices 42 ($N_{as}$) is preferably set to a pre-determined value, which may be interactively modified by the user according to the shape of the aneurysm 26 to be segmented by editing the corresponding input field using the interaction device 24. $N_{as}$ is illustratively set by default to four (4) angular slices 42 for aneurysms 26 of generally circular shape but it may be increased for aneurysms 26 with a less regular shape, e.g. when the aneurysm 26 is very off-centre. In the latter case, the number of angular slices 42 is increased to create more cross-sections around the more irregular areas of the aneurysm 26, thereby better defining and more accurately representing it. The value of $N_{as}$ defines the spacing step (in degrees) for the angular positioning θ of the slices 42. This spacing step may be computed as follows:

$$\text{Spacing step} = (180)/ N_{as} \hspace{4cm} (2)$$

As seen in Figure 6a for example, $N_{as}$ is set to four (4), thereby defining angular slices 42 regularly spaced by a spacing step of 45 degrees. The corresponding angular positions θ of the slices 42 are illustratively then 0, 45, 90, and 135 degrees. The user may further edit the configuration, position, and number of the angular slices 42 (or half-slices 42'), leading to angular slices 42 which are irregularly spaced. Such irregular spacing of the angular slices 42 may be desirable to better define the volume under inspection, especially when the latter is not perfectly circular, in which case more slices 42 should be introduced, as discussed herein above. As shown in Figure 7a and Figure 7b, the angular position θ of a slice 42 may be edited with the user interaction device 24 by mouse click and drag, thus changing the position of the selected angular slice 42. In Figure 7a, in order to avoid an artefact 44, the angular position θ of a full slice 42 is moved while in Figure 7b only a half-slice 42' is edited by mouse drag. Similarly, a selected slice 42 (or half-slice) can be removed and new slices (or half-slices) added by mouse click and drag.

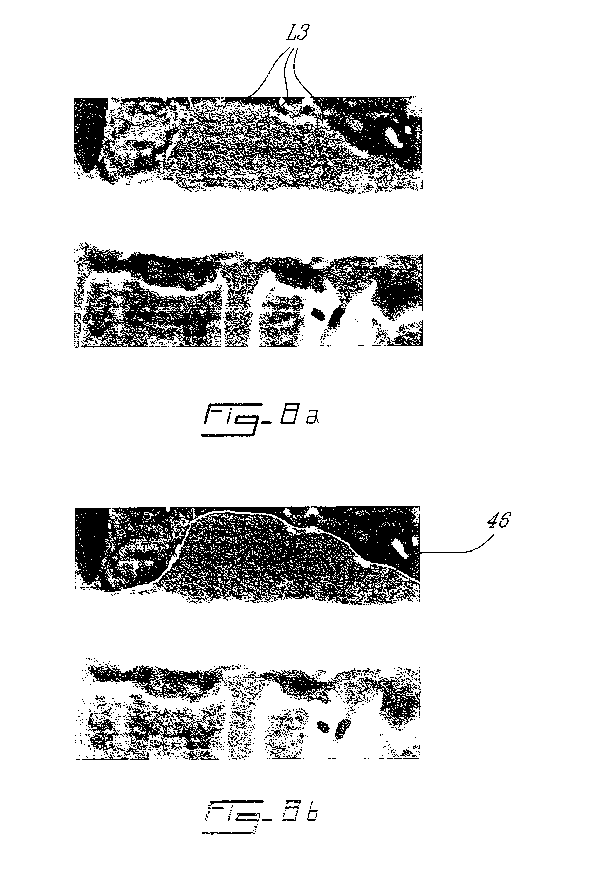

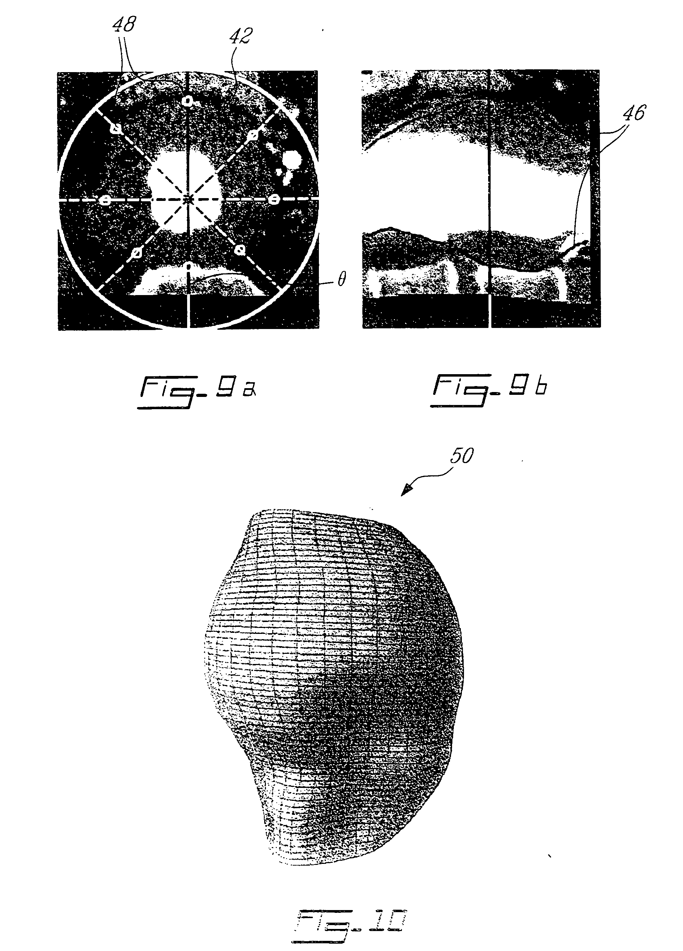

Now referring to Figure 8a, Figure 8b, Figure 9a and Figure 9b in addition to Figure 2, Figure 3 and Figure 7, once the configuration of the slices 42 has been validated by the user, the latter may proceed with the segmentation (110) of the aneurysm boundaries. For this purpose, the user illustratively uses an active contour method to segment the outer aneurysm wall 30 in the angular slices 42 defined beforehand. This method is an iterative energy-minimizer method, which is based on the rigidity of the deformable contour. Livewire segmentation may also be used as a segmentation method. In this case, regions of interest are extracted based on Dijkstra's algorithm by calculation of a smallest cost path between selected landmarks. Another segmentation approach that can be used is active-shape contour, which specifies the shape of the segmented boundary curve for a particular type of objects a *priori,* based on statistics of a set of images and measurements of the relevant area. This enables natural inclusion of anatomical knowledge into the segmentation process. Indeed, the borders in a particular anatomical scene are characterized by discrete samples at the contours, with these points being situated at selected landmarks characteristic for every image of the same scene, e.g. typical corners, bays or protrusions, holes, and blood vessel branching. The selection of a set of such landmarks is carried out in preparation of the segmentation procedure. Depending on the image character, the feature points in the typical image may form one or more closed borders surrounding anatomically meaningful means.

As illustrated in Figure 8a and Figure 8b, using active-shape contour segmentation, the user interactively places several landmarks L3 (Figure 8a) near the aneurysm wall 30 by mouse click, thus generating automatic segmentation of the aneurysm boundary 46 (Figure 8b). The user may further control the quality of the segmentation on the axial view (112). The segmented boundary 46 may be locally edited to correct the position of some points as needed. As illustrated in Figure 9a, the intersection between the observed axial plane and the segmented aneurysm boundaries 46 is represented by points 48 located on the respective angular slices 42. The user may push or pull a local region on all boundary curves 46 (Figure 8b) and thus edit the latter using specific mouse-defined functions. After manual deformation, the boundary curves 46 will be automatically optimized by local active contour deformation. Alternatively, the segmentation process may be applied on images illustrated in Figure 7a and Figure 7b, such images being substantially perpendicular to the ones illustrated in Figure 8a and Figure 8b. In this case, the user similarly initializes the active contour interactively as a closed contour on several slices, the active contour being initialized either by placing successive markers, such as the landmarks mentioned herein above, or by positioning a parametrical model, such as a circle or ellipse, subsequently transformed and optimized in the image space. Still, although active-shape contour has been used as a segmentation approach, it will be apparent to one skilled in the art that other methods, such as parametric and geometric flexible contour algorithms, may be used.

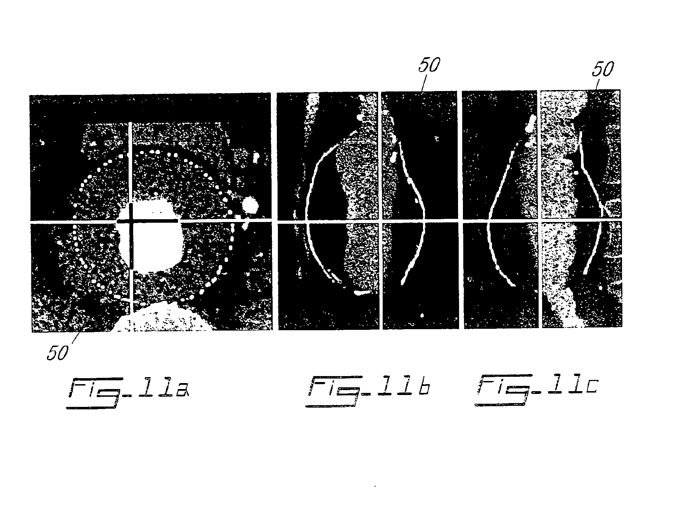

Referring now to Figure 10, Figure 11 a, Figure 11 b and Figure 11 a in addition to Figure 2 and Figure 3, following quality control and correction at 112, a 3D parametric surface representation 50 of the aneurysm wall 30 is automatically computed at 114 (although one skilled in the art would recognize that other visualization techniques are possible). This 3D surface mesh model 50 (illustrated in Figure 10) is then back-projected in the initial image space (i.e. the native DICOM images), resliced and represented in axial (Figure 11a), sagittal (Figure 11 b) and coronal (Figure 11c) views. From the 3D wall model 50, it is then possible to proceed with quantification of the aneurysm parameters (116). At this point, the geometrical centreline (represented by the dashed line associated with reference B in Figure 3) of the aneurysm 26, which passes through the centre of the aneurysm 26 and whose points are all at equidistance from the aneurysm wall 30, is computed. This geometrical centreline B, which differs from the minimum-curvature path A described herein above and used to define cross-sections, is used to compute the value of the maximum diameter $D_{max}$ of the aneurysm 26. Indeed, upon extraction of the centreline B, the 3D wall model 50 is automatically resliced by cross-section planes defined along this new centreline B. The maximal distance between all points on the 3D wall model 50 is then computed in each centreline-defined cross-section, illustratively using the following pseudo-code:

```
All_Pts = matrix(M,N,3)
 for j=1, N do begin
 X = All_Pts(*,j,1 )
 Y = All_Pts(*,j,2)
 Z = All_Pts(*,j,3)
 for i=1, M do begin
        diam = max(sqrt(((x[i]-x))^2 +((y[i]-y))^2 +((z[i]-z))^2))
        aThrombusALLMaxDiameters[j,i] = diam
 endfor
 endfor
 with All_Pts = matrix of all data points on the parametric 3D model;
 diam = maximum diameter mapped at a given point of the 3D model.
```

The final matrix aThrombusALLMaxDiameters holds the value of $D_{max}$ for each point of the 3D aneurysm wall model 50. Similarly, other attributes or components of the aneurysm 26, such as the thickness of the thrombus 40, lumen 38, wall 30, calcifications and plaque (not shown), can be measured in order to monitor changes over time.

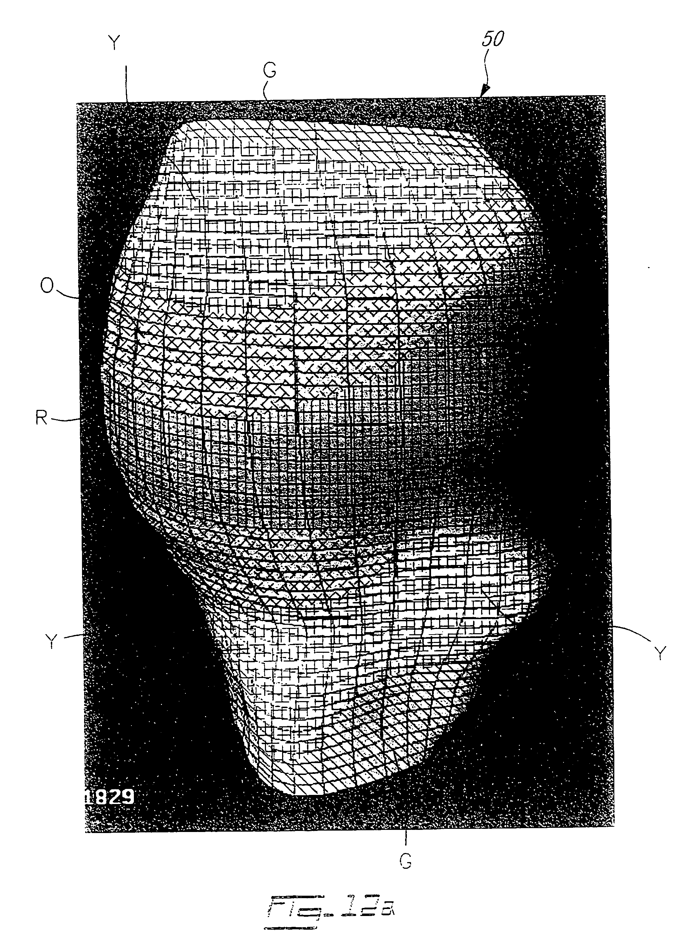

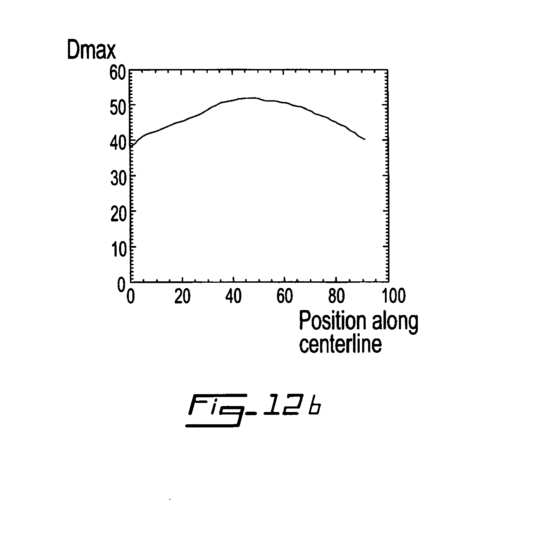

Referring now to Figure 12a and Figure 12b in addition to Figure 2 and Figure 3, in order to provide clear information regarding the local parameter values of the aneurysm 26, the 3D surface wall model 50 is augmented with a coding, such as colour-coding, shading, hatching, or the like. A combination of hatching, colour and letter coding (with B for blue, C for cyan, G for green, Y for yellow, O for orange and R for red) is shown in Figure 12a for illustrative purposes only, although a person of skill in the art will appreciate that any other suitable coding may be used to represent the measured parameters. Illustratively, the $D_{max}$ value is mapped on the 3D model 50 using a colour scale, for example one which varies from blue to red or the like to represent increasing values of $D_{max}$. Alternatively, $D_{max}$ may be represented for each cross-section along the centreline B, as shown in Figure 12b. This representation advantageously shows the $D_{max}$ profile along the centreline B in a two-dimensional (2D) curve. The maximal value on the curve is therefore the sought global value of $D_{max}$, which can be used as a diagnostic measure of the aneurysm 26. For a patient having undergone two clinical imaging exams at times t1 and t2, and thus for two respective image sets $IS_1$ and $IS_2$, two values $D_{max1}$ and $D_{max2}$ of the maximal diameter are computed for each image set. The change in the maximal diameter of the aneurysm 26 over time is then computed as the difference between $D_{max1}$ and $D_{max2}$. At 118, once the aneurysm parameters have been quantified, the results are stored in the database 12 for subsequent review. This allows patient monitoring and follow up by enabling the study of the expansion rate of the $D_{max}$ parameter (and similarly other attributes of the aneurysm 26 mentioned herein above) in the long run.

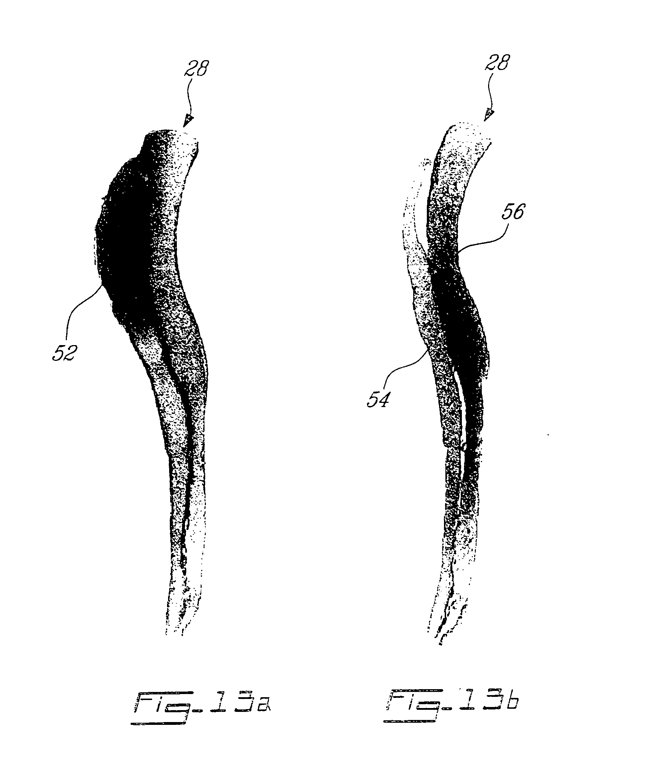

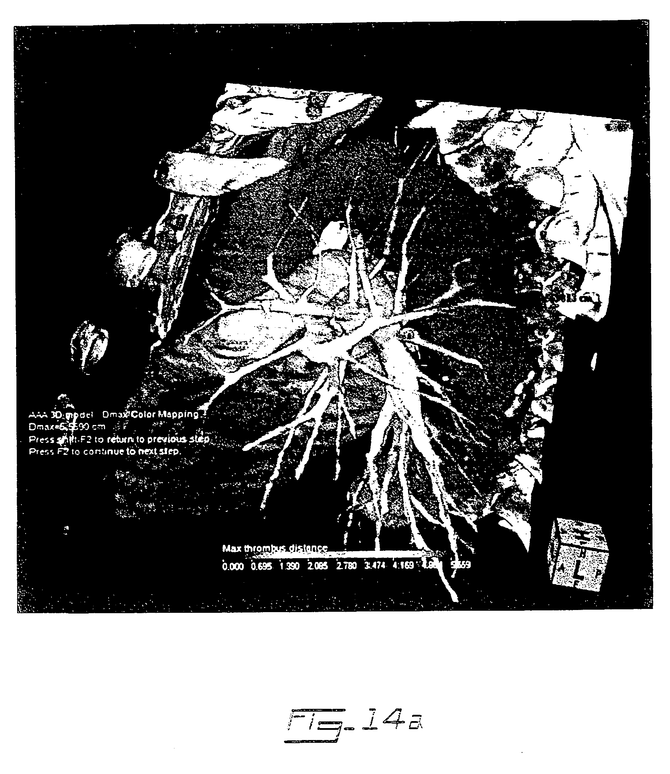

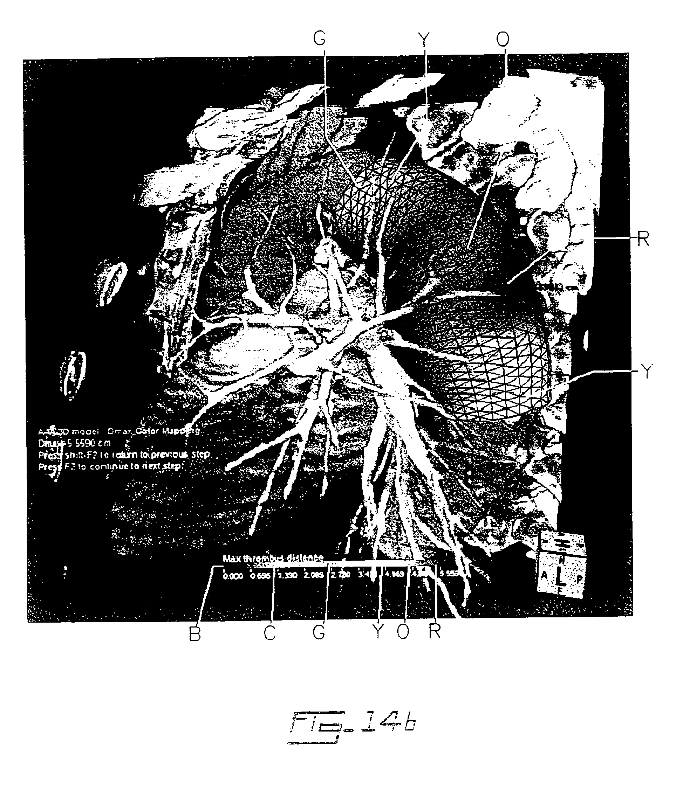

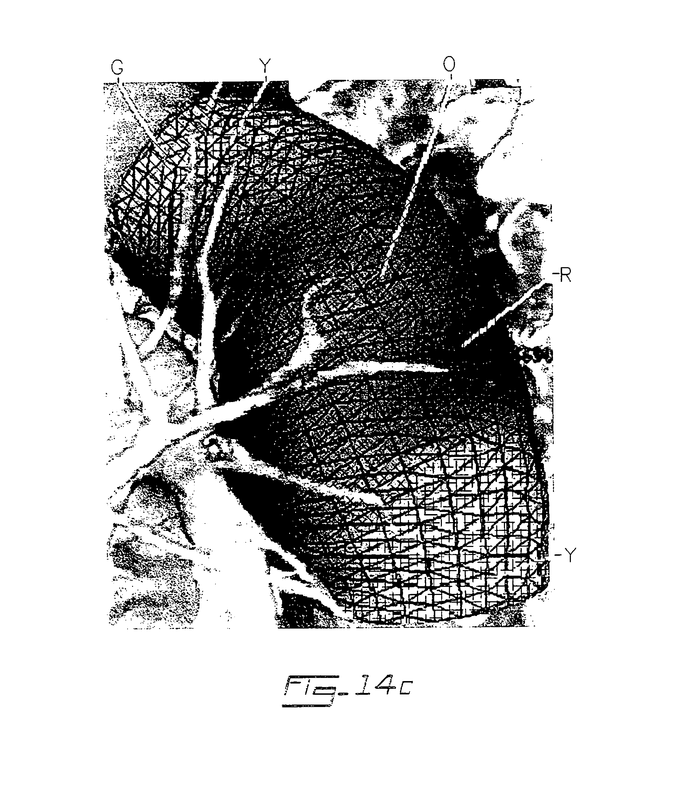

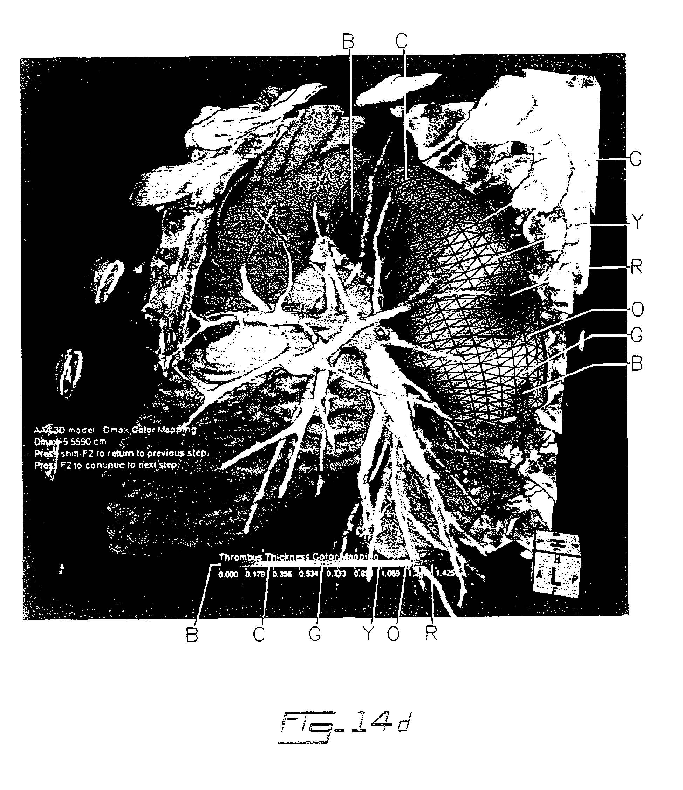

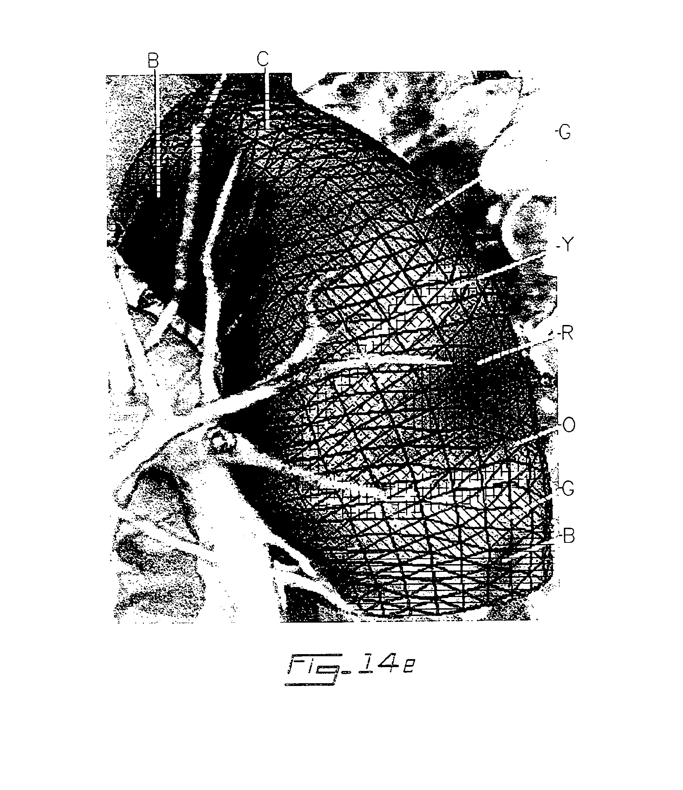

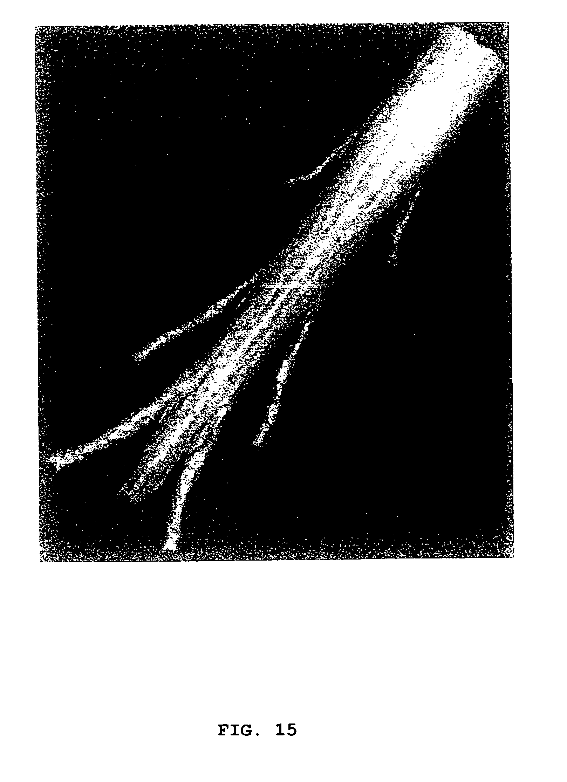

Referring now to Figure 13a, Figure 13b, Figure 14a, Figure 14b, Figure 14c, Figure 14d, Figure 14e, and Figure 15, the present invention can be used for a plurality of applications. For example, the segmentation method illustratively allows to distinguish the volume of the false thrombus 52 (Figure 13a), i.e. the abnormal channel within the wall of the aorta 28, from the volume of the pathological components 54 and 56 (Figure 13b) of the aorta lumen (reference 38 in Figure 3), which are due to aortic dissection, a tear in the wall of the aorta 28 that causes blood to flow between the layers of the aortic wall and to force the layers apart. In this case, the aorta 28 is illustratively automatically segmented from the aortic arch to the iliac bifurcation (both not shown). Also, as mentioned previously, the segmentation process described herein above can be applied to anatomical structures other than abdominal aortic aneurysms, such as thoracic aortic aneurysms for example. This is illustrated in Figure 14a, which, in the case of a thoracic aortic aneurysm, shows the segmentation of the aorta lumen 38. Figure 14b and Figure 14c further show the segmentation of the thrombus (reference 40 in Figure 3) and the mapping of the $D_{max}$ value on the 3D model (reference 50 in Figure 10) using coding, illustratively hatching, although it will be apparent to a person skilled in the art that a colour scale or the like could be used without departing from the scope of the present invention, as discussed herein above with reference to Figure 12a and Figure 12b. Similarly, Figure 14d and Figure 14e illustrate the segmentation of the thrombus 40 and the mapping of the thrombus thickness on the 3D model 50 using a suitable coding. Moreover, Figure 15 illustrates the application of the method of the present invention for segmentation of a cat's spinal cord (not shown).

When two or more sets of image data from one region are acquired at different times, using different imaging modalities, or for different patient orientations, it is desirable for them to be co-registered before segmentation. This will ensure that corresponding image features are substantially identically positioned in the matrices of image data and thus spatially consistent. Indeed, the imaging geometry for each of the images may be different due to possibly different physical properties and distortions inherent to different modalities. Also, the imaged scene itself may change between taking individual images due to patient movements, and/or physiological or pathological deformations of soft tissues. Ideally, a particular point in each of the registered images would correspond to the same unique spatial position in the imaged object, e.g. a patient. Registration thus transforms the images geometrically, in order to compensate for the distortions and fulfil the consistency condition. Typically, one of the images, which may be considered undistorted, is taken as the reference (base) image. The process of registration illustratively uses a geometrical transformation controlled by a parameter vector that transforms one image into a transformed image, which is then laid on (i.e. spatially identified with) the other (base) image so that both images can be compared. A degree of accuracy and precision is required when registering medical images as imprecise registration leads to a loss of resolution or to artefacts in the combined (fused) images, viihile unreliable and possibly false registration may cause misinterpretation of the fused image (or of the information obtained by fusion), with possibly fatal consequences.

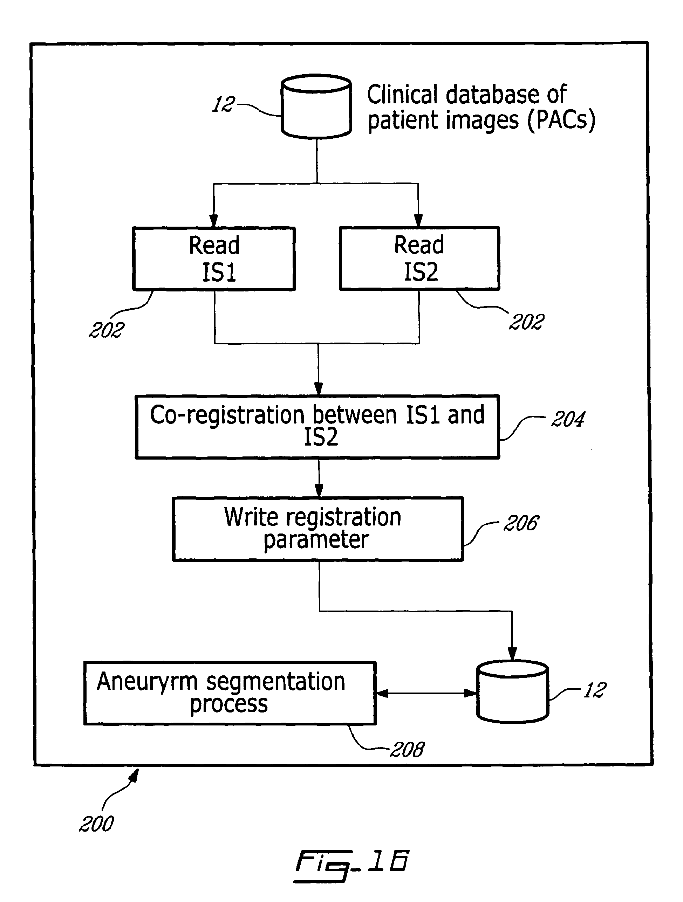

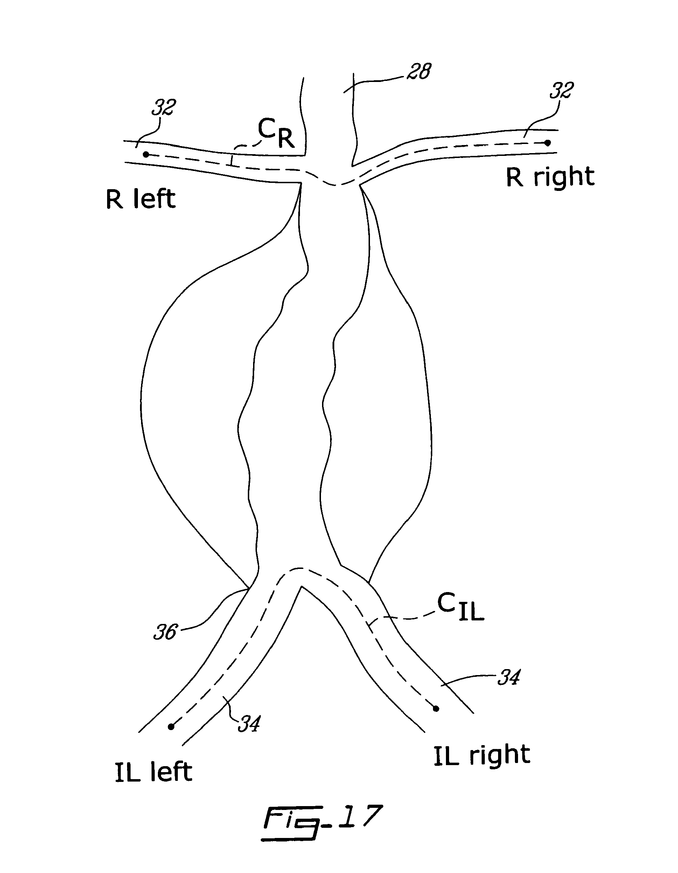

Referring now to Figure 16 and Figure 17 in addition to Figure 1, an image registration method 200 according to the present invention will now be described. In order to co-register two image sets $IS_1$ and $IS_2$ (acquired for the same patient at times t1 and t2), which have been read by the imaging software 20 at 202, four vascular landmarks are initialized in each image set (204). This can be done, for example as illustrated in Figure 17 (for a single image set), by a user defining (preferably in MPR view) two landmarks, $R_{left}$ and $R_{right}$, in the left and right renal arteries 32 respectively and two other landmarks, $IL_{left}$ and $IL_{right}$, in the left and right iliac arteries 34 respectively, after the bifurcation 36 of the aorta 28. After landmark initialization, vascular centreline-paths are extracted from the landmarks. Illustratively, a first vessel centreline-path, the renal path $C_R$, is computed from $R_{right}$ to $R_{left}$ while a second vessel centreline-path, the iliac path $C_{IL}$, is computed from $IL_{right}$ to $IL_{left}$. Similarly to 106 described herein above with reference to Figure 2, these centreline paths $C_R$ and $C_{IL}$ are obtained illustratively using the Dijkstra shortest path algorithm on the images smoothed by a Gaussian filter. The vessel curves thus obtained are represented as ordered discrete points defined in the image coordinate system. As will now be apparent to a person of skill in the art, more than two such vessel curves may be extracted from the initialized landmarks $R_{right}$, $R_{left}$, $IL_{right}$ and $IL_{left}$, resulting in more accurate registration of the image sets $IS_1$ and $IS_2$. For example, two additional centreline paths may be computed from $R_{left}$ to $IL_{right}$ and $R_{right}$ to $IL_{left}$ respectively.

Still referring to Figure 16 and Figure 17 in addition to Figure 1, the similarity criteria between the renal paths $C_R$ and iliac paths $C_{IL}$ extracted from each image set $IS_1$ and $IS_2$ are then identified. Similarity criteria, which serve to evaluate the resemblance of two (and possibly more) images or their areas, must be evaluated when matching two or more images via geometrical transformations, as is the case of image registration. For this purpose, it is desirable to use a method independent of location, rotation and scale. The curve signature of each centreline path $C_R$ and $C_{IL}$ can thus be represented by its local tangent, curvature and torsion. More specifically, the curve arc-length is illustratively normalized and the curve signature is computed, followed by signature correlation between the two renal paths $C_R$ and the two iliac paths $C_{IL}$. Point to point association is then achieved by maximum correlation detection, thus leading to 3D registration between paired points. As a result, an affine transformation matrix with three (3) rotation and three (3) translation parameters is illustratively obtained. These registration parameters are stored in the database 12 at 206 and the transformation is applied to one of the image sets, i.e. either $IS_1$ or $IS_2$, in order to co-register it with the other image set.

The above registration process may be further improved using an image-based processes such as mutual information algorithms. Mutual information, which proves to be a good criterion of similarity, is defined as the difference between the sum of information in individual images and the joint information in the union. Use of the mutual information algorithm results in masking the image sets by a weighted function that enables an image volume element (voxel) near the centreline and disables the others, thus showing how much the a *priori* information content of one image is changed by obtaining the knowledge of the other image.

Referring now to Figure 2 and Figure 3 in addition to Figure 16, following co-registration of the image sets $IS_1$ and $IS_2$, the segmentation process (208) may proceed as described above, with a minimum-curvature path A being extracted in a similar manner as in 106. However, since the images have been co-registered before they are segmented, the segmentation algorithm will use the pair of co-registered image sets together to ensure that the extracted minimum-curvature path is defined inside both lumens of the two superimposed image sets. The results obtained with co-registered images are more efficient since the real changes in volume, surface, and thickness may be illustratively computed and mapped in 3D, as the two image sets $IS_1$ and $IS_2$ are superimposed in the same geometrical reference frame. Moreover, local and global changes in geometry and topology of the aneurysm may be obtained for the two image sets.

As will now be apparent to one skilled in the art, the approach described herein is efficient whether contrast agents have been used or not. Contrast agents are not used during all clinical imaging exams, as it is preferable to avoid their use in some cases, such as when the patient under observation is suffering from renal failure. If no contrast agent has been used, although the lumen 38 (Figure 3) will potentially have the same gray level distribution as the thrombus 40, it is still possible to quantify the maximum diameter as well as the aneurysm volume using the method described herein above. More importantly, the diagnostic tool of the present invention achieves fast and accurate results with a high level of reproducibility. The segmentation may therefore be performed in a standardized manner by technicians, thus leading to time savings for doctors and other clinicians who only need to be involved in the subsequent review processes.

Although the present invention has been described hereinabove by way of specific embodiments thereof, it can be modified, without departing from the invention as defined in the appended claims.

## Claims

1. A method (100) for visualizing an anatomy of a region of interest of a tubular-shaped organ on a display (22), the method comprising:

   acquiring (102) a three-dimensional image of the anatomy of the tubular shaped organ in the region of interest at a first point in time, and **characterized by**:

   extracting (106) a plurality of discrete points from said three-dimensional image defining a minimum-curvature path within the tubular-shaped organ;
   interpolating (108) a set of cross-sectional images along planes substantially perpendicular to a tangent vector of said minimum-curvature path at each of said plurality of discrete points;
   delimiting a segmented area corresponding to the region of interest of the tubular-shaped organ in each of said set of cross-sectional images;
   rendering (114) a three-dimensional surface representation of the region of interest from said delimited set of cross-sectional images; and
   displaying said rendered three-dimensional surface representation on the display.

2. The method of claim 1, **characterized in that** said three-dimensional image is comprised of a plurality of image slices, preferably further comprising positioning at least two reference markers in said image slices, wherein said reference markers preferably comprise a first reference marker and a second reference marker, wherein said minimum-curvature path connects said reference markers, and wherein said positioning reference markers in said image slices is preferably performed in Multi-Planar Reformatting (MPR) view.

3. The method of claim 1, **characterized in that** the tubular-shaped organ has a longitudinal axis and further wherein said acquiring successive image slices comprises taking each one of said image slices along a plane substantially perpendicular to said longitudinal axis.

4. The method of claim 1, **characterized in that** said acquiring successive image slices comprises using an image modality selected from the group consisting of Computed Tomography angiography and Magnetic Resonance Imaging angiography.

5. The method of claim 1, **characterized in that** said extracting a plurality of discrete points comprises:

   obtaining a plurality of discrete point coordinates defining a lowest-cost path between said reference markers using Dijkstra's algorithm;
   deriving gray-level values of each one of said plurality of discrete point coordinates;
   computing from said derived gray-level values fuzzy image representations of said acquired image slices;
   computing a distance map representative of a distance from a discrete point in each one of said fuzzy image representations to an adjacent obstacle point in said one fuzzy image representation; and
   computing said minimum-curvature path from said distance map, wherein said minimum-curvature path is preferably computed from said distance map by applying back propagation from said second reference marker to said first reference marker using an optimization algorithm, preferably a gradient descent algorithm;
   wherein said computing a distance map preferably comprises applying a fast-marching algorithm based on propagation of a wave front from said first reference marker to said second reference marker.

**6.** The method of claim 1, **characterized in that** said interpolating cross-sectional images comprises defining a Frenet reference frame at a first one of said reference markers, and, for a successive one of said discrete points along said minimum-curvature path, recomputing said Frenet reference frame and propagating said recomputed Frenet reference frame to said successive one of said discrete points.

**7.** The method of claim 1, **characterized in that** said segmented area is delimited in an axial representation and in an angular representation of each of said cross-sectional images, wherein said angular representation preferably comprises a plurality of angular slices of each of said cross-sectional images acquired at a plurality of angles around said minimum-curvature path, and further wherein a positioning and a number of said angular slices is preferably selected to accurately define the region of interest.

**8.** The method of claim 1, **characterized in that** said delimiting a segmented area is performed using a method selected from a group consisting of active-shape contour segmentation, parametric flexible contour segmentation, geometric flexible contour segmentation, and livewire segmentation.

**9.** The method of claim 1, further comprising quantifying an attribute of the region of interest from said three-dimensional surface representation, preferably wherein said attribute of the region of interest is selected from a group consisting of maximal diameter and volume, and augmenting said three-dimensional surface representation with a coding representative of said attribute, preferably wherein said coding is selected from a group consisting of colour, shading and hatching or combinations thereof and wherein quantifying said maximal diameter of the region of interest preferably comprises:

computing a geometrical centreline of the region of interest;
slicing said three-dimensional surface representation by cross-section planes defined along said geometrical centreline to generate a plurality of centreline-defined cross-sections; and
computing a maximal distance between discrete points in each one of said plurality of centreline-defined cross-sections.

**10.** The method of claim 9, **characterized in that** said quantifying an attribute of the region of interest comprises:

acquiring a second three-dimensional image of the anatomy of the tubular shaped organ in the region of interest at a second point in time;
extracting a second plurality of discrete points from said second three-dimensional image, said second points defining a minimum-curvature path within the tubular-shaped organ;
interpolating a second set of cross-sectional images along planes substantially perpendicular to a tangent vector of said minimum-curvature path at each of said second plurality of discrete points;
delimiting a segmented area corresponding to the region of interest of the tubular-shaped organ in each of said second set of cross-sectional images;
rendering a second three-dimensional surface representation of the region of interest from said delimited second set of cross-sectional images;
calculating a difference between said three-dimensional surface representation and said second three-dimensional surface representation; and
augmenting said three-dimensional surface representation with a coding representative of said difference.

**11.** A system for visualizing the anatomy of a region of interest of a tubular-shaped organ, the system comprising:

a scanning device for acquiring a three-dimensional image of the region of interest of the tubular shaped organ;
a database (12) connected to said scanning device for storing said acquired three-dimensional image; and
a workstation (14) connected to said database for retrieving said stored image, said workstation comprising:

a display (22);
a user interface (24); and
an image processor (18, 20);
**characterized in that** responsive to said commands from said user interface, said image processor extracts from said image a plurality of discrete points defining a minimum-curvature path within the region of interest of the tubular-shaped organ, interpolates a set of cross-sectional images along planes substantially perpendicular to a tangent vector of said minimum-curvature path at each of said plurality of discrete points, delimits a segmented area corresponding to the region of interest of the tubular-shaped organ in each of said set of cross-sectional images, computes a three-dimensional surface representation of the region of interest from said delimited set of cross-sectional images and displays said computed three-dimensional surface representation on said display.

12. A computer program storage medium readable by a computing system and encoding a computer program of instructions for executing a computer process for visualizing the anatomy of a region of interest of a tubular-shaped organ, the computer process comprising:

acquiring a three-dimensional image of the anatomy of the tubular shaped organ in the region of interest, and **characterized by**:

extracting from said three-dimensional image a plurality of discrete points defining a minimum-curvature path within the tubular-shaped organ;
interpolating a set of cross-sectional images along planes substantially perpendicular to a tangent vector of said minimum-curvature path at each of said discrete points;
delimiting a segmented area corresponding to the region of interest of the tubular-shaped organ in each of said set of cross-sectional images;
computing a three-dimensional surface representation of the region of interest from said delimited set of cross-sectional images; and
displaying said rendered three-dimensional surface representation on the display.

## Patentansprüche

1. Verfahren (100) zum Sichtbarmachen einer Anatomie eines Bereichs von Interesse eines röhrenförmigen Organs auf einer Anzeige (22), **dadurch gekennzeichnet, dass** das Verfahren Folgendes aufweist:

ein Beschaffen (102) eines dreidimensionales Bilds der Anatomie des röhrenförmigen Organs in dem Bereich von Interesse zu einem ersten Zeitpunkt;
ein Extrahieren (106) einer Mehrzahl von diskreten Punkten aus dem dreidimensionalen Bild, die einen Weg minimaler Krümmung innerhalb des röhrenförmigen Organs definieren;
ein Interpolieren (108) eines Satzes von Querschnittsbildern entlang Ebenen im Wesentlichen senkrecht zu einem Tangentenvektor des Wegs minimaler Krümmung an jedem der Mehrzahl von diskreten Punkten;
ein Begrenzen eines segmentierten Gebiets entsprechend dem Bereich von Interesse des röhrenförmigen Organs in jedem des Satzes von Querschnittsbildern;
ein Rendern (114) einer dreidimensionale Oberflächendarstellung des Bereichs von Interesse aus dem begrenzten Satz von Querschnittsbildern; und
ein Anzeigen der gerenderten dreidimensionalen Oberflächendarstellung auf der Anzeige.

2. Verfahren nach Anspruch 1, **dadurch gekennzeichnet, dass** das dreidimensionale Bild eine Mehrzahl von Bildschichten oder Bildscheiben aufweist, vorzugsweise ferner ein Positionieren von wenigstens zwei Referenzmarkierungen in den Bildschichten oder Bildscheiben aufweisend, wobei die Referenzmarkierungen vorzugsweise eine erste Referenzmarkierung und eine zweite Referenzmarkierung aufweisen, wobei der Weg minimaler Krümmung die Referenzmarkierungen verbindet, und
wobei das das Positionieren der Referenzmarkierungen in den Bildschichten oder Bildscheiben vorzugsweise in einer Multi-Planar-Neuformatierungs (MPR = Multi-Planar Reformatting)- Ansicht durchgeführt wird.

3. Verfahren nach Anspruch 1, **dadurch gekennzeichnet, dass** das röhrenförmige Organ eine Längsachse hat, wobei ferner das Beschaffen der aufeinander folgenden Bildschichten oder Bildscheiben ein Aufnehmen jeder der Bildschichten oder Bildscheiben entlang einer Ebene, die im wesentlichen senkrecht zu der Längsachse ist, aufweist.

4. Verfahren nach Anspruch 1, **dadurch gekennzeichnet, dass** das Beschaffen der aufeinander folgenden Bildschichten oder Bildscheiben eine Verwendung einer Bildmodalität aufweist, die aus der Gruppe ausgewählt ist, die aus Computertomographie-Angiographie und Magnetresonanz-Abbildungs- Angiographie besteht.

5. Verfahren nach Anspruch 1, **dadurch gekennzeichnet, dass** das Extrahieren einer Mehrzahl von diskreten Punkten Folgendes aufweist:

ein Erhalten einer Mehrzahl von diskreten Punkt-Koordinaten, die einen Weg niedrigster Kosten zwischen den Referenzmarkierungen definiert, unter Verwendung eines Dijkstra-Algorithmus;

ein Ableiten von Graupegelwerten einer jeden der Mehrzahl von diskreten Punkt-Koordinaten;

ein Berechnen von Fuzzy-Bilddarstellungen der beschafften Bildschichten oder Bildscheiben aus den abgeleiteten Graupegelwerten;

ein Berechnen einer Abstands-Karte, die für einen Abstand von einem diskreten Punkt in jeder einen der Fuzzy-Bilddarstellungen zu einem benachbarten Hindernispunkt in der einen Fuzzy-Bilddarstellung repräsentativ ist; und

ein Berechnen des Wegs minimaler Krümmung aus der Abstands-Karte, wobei der Weg minimaler Krümmung vorzugsweise aus der Abstands-Karte berechnet wird durch ein Anwenden einer Rückwärts-Ausbreitung oder Back-Propagation von der zweiten Referenzmarkierung zu der ersten Referenzmarkierung unter Verwendung eines Optimierungsalgorithmus', vorzugsweise eines Gradienten-Abnahme-Algorithmus;

wobei das Berechnen der Abstands-Karte vorzugsweise ein Anwenden eines Fast-Marching-Algorithmus basierend auf einer Ausbreitung einer Wellenfront von der ersten Referenzmarkierung zu der zweiten Referenzmarkierung aufweist.

6.  Verfahren nach Anspruch 1, **dadurch gekennzeichnet, dass** das Interpolieren von Querschnittsbildern ein Definieren eines Frenet-Bezugsrahmens an einer ersten einen der Referenzmarkierungen und für einen nachfolgenden einen der diskreten Punkte entlang des Wegs minimaler Krümmung ein Neu-Errechnen des Frenet-Bezugsrahmens und ein Propagieren des neuberechneten Frenet-Bezugsrahmens auf den nachfolgenden einen der diskreten Punkte aufweist.

7.  Verfahren nach Anspruch 1, **dadurch gekennzeichnet, dass** das segmentierte Gebiet in einer axialen Darstellung und in einer Winkeldarstellung jedes der Querschnittsbilder begrenzt ist, wobei die Winkeldarstellung vorzugsweise eine Mehrzahl von Winkelscheiben oder Winkelschichten jedes der Querschnittsbilder aufweist, welche unter einer Mehrzahl von Winkeln um den Weg minimaler Krümmung beschafft werden, und wobei ferner eine Positionierung und eine Anzahl der Winkelscheiben oder Winkelschichten vorzugsweise ausgewählt ist, so dass sie den Bereich von Interesse genau definieren.

8.  Verfahren nach Anspruch 1, **dadurch gekennzeichnet, dass** das Begrenzen eines segmentierten Gebiets unter Verwendung eines Verfahrens durchgeführt wird, das aus einer Gruppe ausgewählt ist, die aus einer aktiv-Form-Kontursegmentierung, einer parametrisch flexiblen Kontursegmentierung, einer geometrisch flexiblen Kontursegmentierung und einer livewire Segmentierung besteht.

9.  Verfahren nach Anspruch 1, ferner umfassend ein Quantifizieren eines Attributs des Bereichs von Interesse aus der dreidimensionalen Oberflächendarstellung, wobei vorzugsweise das Attribut des Bereichs von Interesse aus einer Gruppe ausgewählt ist, die aus einem maximalen Durchmesser und Volumen besteht, und ein Erweitern der dreidimensionalen Oberflächendarstellung um eine Aufschlüsselung, die für das Attribut repräsentativ ist, wobei die Aufschlüsselung vorzugsweise aus einer Gruppe ausgewählt ist, die aus einer Farbe, einer Schattierung und einer Schraffur oder Kombinationen davon besteht, und wobei das Quantifizieren des maximalen Durchmessers des Bereichs von Interesse vorzugsweise Folgendes aufweist:

ein Berechnen einer geometrischen Mittellinie des Bereichs von Interesse;

ein Aufschneiden der dreidimensionalen Oberflächendarstellung durch Querschnittebenen, die entlang der geometrischen Mittellinie definiert sind, um eine Mehrzahl von Mittellinien-definierten Querschnitten zu erzeugen; und

ein Berechnen eines maximalen Abstandes zwischen diskreten Punkten in jedem der Mehrzahl von Mittellinien-definierten Querschnitten.

10.  Verfahren nach Anspruch 9, **dadurch gekennzeichnet, dass** das Quantifizieren eines Attributs des Bereichs von Interesse Folgendes aufweist:

ein Beschaffen eines zweiten dreidimensionalen Bildes der Anatomie des röhrenförmigen Organs in dem Bereich von Interesse zu einem zweiten Zeitpunkt;

ein Extrahieren einer zweiten Mehrzahl von diskreten Punkten aus dem zweiten dreidimensionalen Bild, wobei die zweiten Punkte einen Weg minimaler Krümmung innerhalb des röhrenförmigen Organs definieren;

ein Interpolieren eines zweiten Satzes von Querschnittsbildern entlang Ebenen im Wesentlichen senkrecht zu einem Tangentenvektor des Wegs minimaler Krümmung an jedem der zweiten Mehrzahl von diskreten Punkten;

ein Begrenzen eines segmentierten Gebiets entsprechend dem Bereich von Interesse des röhrenförmigen Organs in jedem des zweiten Satzes von Querschnittsbildern;

ein Rendern einer zweiten dreidimensionalen Oberflächendarstellung des Bereichs von Interesse aus dem begrenzten zweiten Satz von Querschnittsbildern;

ein Berechnen einer Differenz zwischen der dreidimensionalen Oberflächendarstellung und der zweiten dreidimensionalen Oberflächendarstellung; und

ein Erweitern der dreidimensionalen Oberflächendarstellung um eine Aufschlüsselung, die für die Differenz repräsentativ ist.

11. System zum Sichtbarmachen der Anatomie eines Bereichs von Interesse eines röhrenförmigen Organs, wobei das System Folgendes aufweist:

eine Abtastvorrichtung zum beschaffen eines dreidimensionalen Bildes des Bereichs von Interesse des röhrenförmigen Organs;

eine Datenbank (12), die mit der Abtastvorrichtung verbunden ist, zum Speichern des beschafften dreidimensionalen Bildes, und

eine Workstation (14), die mit der Datenbank verbunden ist, zum Abrufen des gespeicherten Bildes, wobei die Workstation Folgendes aufweist:

eine Anzeige (22);
eine Benutzerschnittstelle (24); und
einen Bildprozessor (18, 20);

**dadurch gekennzeichnet, dass** in Antwort auf die Befehle von der Benutzerschnittstelle der Bildprozessor aus dem Bild eine Mehrzahl von diskreten Punkten extrahiert, die einen Weg minimaler Krümmung innerhalb des Bereichs von Interesse des röhrenförmigen Organs definiert,einen Satz von Querschnittsbildern entlang Ebenen im Wesentlichen senkrecht zu einem Tangentenvektor des Weges minimaler Krümmung an jedem der Mehrzahl von diskreten Punkten interpoliert, einen Segmentbereich entsprechend dem Bereich von Interesse des röhrenförmigen Organs in jedem des Satzes von Querschnittsbildern begrenzt, eine dreidimensionale Oberflächendarstellung des Bereichs von Interesse aus dem begrenzten Satz von Querschnittsbildern berechnet und die berechnete dreidimensionale Oberflächendarstellung auf der Anzeige anzeigt.

12. Computerprogramm-Speichermedium, das von einem Computersystem lesbar ist und ein Computerprogramm aus Befehlen zum Ausführen eines Computervorgangs zum Sichtbarmachen der Anatomie eines Bereichs von Interesse eines röhrenförmigen Organs kodiert, **dadurch gekennzeichnet, dass** der Computervorgang Folgendes aufweist:

ein Beschaffen eines dreidimensionales Bilds der Anatomie des röhrenförmigen Organs in dem Bereich von Interesse;

ein Extrahieren einer Mehrzahl von diskreten Punkten aus dem dreidimensionalen Bild, die einen Weg minimaler Krümmung innerhalb des röhrenförmigen Organs definieren;

ein Interpolieren eines Satzes von Querschnittsbildern entlang Ebenen im Wesentlichen senkrecht zu einem Tangentenvektor des Wegs minimaler Krümmung an jedem der diskreten Punkten;

ein Begrenzen eines segmentierten Gebiets entsprechend dem Bereich von Interesse des röhrenförmigen Organs in jedem des Satzes von Querschnittsbildern;

ein Berechnen einer dreidimensionale Oberflächendarstellung des Bereichs von Interesse aus dem begrenzten Satz von Querschnittsbildern, und

ein Anzeigen der gerenderten dreidimensionalen Oberflächendarstellung auf der Anzeige.


**Revendications**

1. Procédé (100) de visualisation d'une anatomie d'une région d'intérêt d'un organe de forme tubulaire sur un écran (22), le procédé comprenant :

l'acquisition (102) d'une image en trois dimensions de l'anatomie de l'organe de forme tubulaire dans la région d'intérêt à un premier point dans le temps, et **caractérisé par** :

l'extraction (106) d'une pluralité de points discrets à partir de ladite image en trois dimensions définissant un trajet de courbure minimale à l'intérieur de l'organe de forme tubulaire ;

l'interpolation (108) d'une série d'images en coupe transversale le long de plans sensiblement perpendiculaires à un vecteur tangent dudit trajet de courbure minimale au niveau de chacun des points discrets de ladite pluralité de points discrets ;

la délimitation d'une zone segmentée correspondant à la région d'intérêt de l'organe de forme tubulaire dans chacune de ladite série d'images en coupe transversale ;

le rendu (114) d'une représentation de surface en trois dimensions de la région d'intérêt à partir de ladite série d'images en coupe transversale délimitées ; et

l'affichage sur l'écran de ladite représentation de surface en trois dimensions rendue.

2. Procédé selon la revendication 1, **caractérisé en ce que** ladite image en trois dimensions est composée d'une pluralité de coupes d'image, de préférence comprenant en outre le positionnement d'au moins deux marqueurs de référence dans lesdites coupes d'image, dans lequel lesdits marqueurs de référence comprennent de préférence un premier marqueur de référence et un second marqueur de référence, dans lequel ledit trajet de courbure minimale relie lesdits marqueurs de référence, et dans lequel ledit positionnement des marqueurs de référence dans lesdites coupes d'image est de préférence réalisé sur une vue de reformatage multiplanaire (MPR).

3. Procédé selon la revendication 1, **caractérisé en ce que** l'organe de forme tubulaire a un axe longitudinal et en outre dans lequel l'acquisition de coupes successives d'image comprend la prise de chacune desdites coupes d'image le long d'un plan sensiblement perpendiculaire au dit axe longitudinal.

4. Procédé selon la revendication 1, **caractérisé en ce que** ladite acquisition de coupes successives d'image comprend l'utilisation d'une modalité d'image choisie dans le groupe constitué de l'angiographie par tomographie par ordinateur et de l'angiographie par imagerie par résonance magnétique.

5. Procédé selon la revendication 1, **caractérisé en ce que** ladite extraction d'une pluralité de points discrets comprend :

l'obtention d'une pluralité de coordonnées de points discrets définissant un trajet moins coûteux entre lesdits marqueurs de référence à l'aide de l'algorithme de Dijkstra ;

le fait de dériver des valeurs de niveau de gris de chacune des coordonnées de points discrets de ladite pluralité de coordonnées de points discrets ;

le calcul, à partir desdites valeurs de niveau de gris obtenues, de représentations d'images floues desdites coupes d'image acquises ;

le calcul d'une carte des distances représentant une distance d'un point discret sur chacune desdites représentations d'images floues jusqu'à un point adjacent formant obstacle sur ladite représentation d'image floue ; et

le calcul dudit trajet de courbure minimale à partir de ladite carte des distances, dans lequel ledit trajet de courbure minimale est de préférence calculé à partir de ladite carte des distances par application d'une propagation arrière dudit second marqueur de référence au dit premier marqueur de référence en utilisant un algorithme d'optimisation, de préférence un algorithme de descente de gradient ;

dans lequel ledit calcul d'une carte des distances comprend de préférence l'application d'un algorithme de Fast Marching en se basant sur la propagation d'un front d'onde dudit premier marqueur de référence au dit second marqueur de référence.

6. Procédé selon la revendication 1, **caractérisé en ce que** ladite interpolation d'images en coupe transversale comprend la définition d'un cadre de référence de Frenet au niveau d'un premier marqueur de référence desdits marqueurs de référence, et, pour l'un successif desdits points discrets sur ledit trajet de courbure minimale, le recalcul dudit cadre de référence de Frenet et la propagation dudit cadre de référence de Frenet recalculé au dit point discret successif desdits points discrets.

7. Procédé selon la revendication 1, **caractérisé en ce que** ladite zone segmentée est délimitée dans une représentation axiale et dans une représentation angulaire de chacune desdites images en coupe transversale, dans lequel ladite représentation angulaire comprend de préférence une pluralité de coupes angulaires de chacune desdites images en coupe transversale acquises à une pluralité d'angles autour dudit trajet de courbure minimale, et en outre dans lequel un positionnement et un certain nombre desdites coupes angulaires sont de préférence choisis pour définir précisément la région d'intérêt.

8. Procédé selon la revendication 1, **caractérisé en ce que** ladite délimitation d'une zone segmentée est réalisée en utilisant un procédé choisi dans un groupe constitué de la segmentation par contour actif, de la segmentation par contour flexible paramétrique, de la segmentation par contour flexible géométrique, et de la segmentation par ciseaux intelligents.

9. Procédé selon la revendication 1, comprenant en outre la quantification d'un attribut de la région d'intérêt à partir de ladite représentation de surface en trois dimensions, de préférence dans lequel ledit attribut de la région d'intérêt est choisi dans un groupe constitué du diamètre maximal et du volume, et l'augmentation de ladite représentation de surface en trois dimensions avec un codage représentatif dudit attribut, de préférence dans lequel ledit codage est choisi dans un groupe constitué de la couleur, de l'ombrage et du hachurage ou de combinaisons de ceux-ci, et dans lequel la quantification dudit diamètre maximal de la région d'intérêt comprend de préférence :

le calcul d'une ligne centrale géométrique de la région d'intérêt ;
la coupe de ladite représentation de surface en trois dimensions selon des plans transversaux définis le long de ladite ligne centrale géométrique pour générer une pluralité de sections transversales définies par la ligne centrale ; et
le calcul d'une distance maximale entre les points discrets dans chacune de ladite pluralité de sections transversales définies par la ligne centrale.

10. Procédé selon la revendication 9, **caractérisé en ce que** ladite quantification d'un attribut de la région d'intérêt comprend :

l'acquisition d'une seconde image en trois dimensions de l'anatomie de l'organe de forme tubulaire dans la région d'intérêt à un second point dans le temps ;
l'extraction d'une seconde pluralité de points discrets de ladite seconde image en trois dimensions, lesdits seconds points définissant un trajet de courbure minimale à l'intérieur de l'organe de forme tubulaire ;
l'interpolation d'une seconde série d'images en coupe transversale le long de plans sensiblement perpendiculaires à un vecteur tangent dudit trajet de courbure minimale au niveau de chacun de ladite seconde pluralité de points discrets ;
la délimitation d'une zone segmentée correspondant à la région d'intérêt de l'organe de forme tubulaire dans chacune de ladite seconde série d'images en coupe transversale ;
le rendu d'une seconde représentation de surface en trois dimensions de la région d'intérêt à partir de ladite seconde série d'images en coupe transversale délimitées ;
le calcul d'une différence entre ladite représentation de surface en trois dimensions et la ladite seconde représentation de surface en trois dimensions ; et
l'augmentation de ladite représentation de surface en trois dimensions avec un codage représentatif de ladite différence.

11. Système de visualisation de l'anatomie d'une région d'intérêt d'un organe de forme tubulaire, le système comprenant :

un dispositif de balayage pour l'acquisition d'une image en trois dimensions de la région d'intérêt de l'organe de forme tubulaire ;
une base de données (12) connectée au dit dispositif de balayage pour stocker ladite image en trois dimensions acquise; et
un poste de travail (14) connecté à ladite base de données pour récupérer ladite image stockée, ledit poste de travail comprenant :

un écran (22) ;
une interface utilisateur (24) ; et
un processeur d'image (18, 20) ;

**caractérisé en ce que** ledit processeur d'image, en réponse auxdites commandes de l'interface utilisateur, extrait de ladite image une pluralité de points discrets définissant un trajet de courbure minimale à l'intérieur de la région d'intérêt de l'organe de forme tubulaire, interpole une série d'images en coupe transversale le long de plans sensiblement perpendiculaires à un vecteur tangent dudit trajet de courbure minimale au niveau de chacun des points discrets de ladite pluralité de points discrets, délimite une zone segmentée correspondant à la région d'intérêt de l'organe de forme tubulaire dans chacune de ladite série d'images en coupe transversale, calcule une représentation de surface en trois dimensions de la région d'intérêt à partir de ladite série d'images en coupe transversale délimitées et affiche sur ledit écran ladite représentation de surface en trois dimensions calculée.

12. Support de stockage d'un programme informatique lisible par un système informatique et codant un programme informatique d'instructions pour l'exécution d'un processus informatique de visualisation de l'anatomie d'une région d'intérêt d'un organe de forme tubulaire, le processus informatique comprenant :

l'acquisition d'une image en trois dimensions de l'anatomie de l'organe de forme tubulaire dans la région d'intérêt et **caractérisé par** :

l'extraction, à partir de ladite image en trois dimensions, d'une pluralité de points discrets définissant un trajet de courbure minimale à l'intérieur de l'organe de forme tubulaire ;
l'interpolation d'une série d'images en coupe transversale le long de plans sensiblement perpendiculaires à un vecteur tangent dudit trajet de courbure minimale au niveau de chacun desdits points discrets ;
la délimitation d'une zone segmentée correspondant à la région d'intérêt de l'organe de forme tubulaire dans chacune de ladite série d'images en coupe transversale ;
le calcul d'une représentation de surface en trois dimensions de la région d'intérêt à partir de ladite série d'images en coupe transversale délimitées ; et
l'affichage sur l'écran de ladite représentation de surface en trois dimensions rendue.

_14_

_18_

PROCESSOR

IMAGING SOFTWARE

_20_

DISPLAY _22_

USER INTERFACE

_24_

_16_

_10_

DATABASE

_12_

Fig-1

*102* → Read angioCT sequence in DICOM format

*104* → Interactive initialisation of two landmarks by mouse click

*106* → Automatic minimal curvature-path extraction

*108* → Automatic cross-section images interpolation

*110* → Supervised aneurysm wall segmentation

*112* → Quality control and correction of the segmentation

*114* → 3D parametric Aneurysm Model computation

*116* → Automatic quantification of aneurysm parameters

*118* → Writing results in Database

Clinical database of patient images (PACs)

*12*

*100*

Fig-2

Fig-3

Fig. 4a

Fig. 4b

A

FIG. 5

Fig. 6a

Fig. 6b

Fig. 7a

Fig. 7b

*L3*

*Fig. 8 a*

*46*

*Fig. 8 b*

*Fig-9a*

*Fig-9b*

*Fig-10*

Fig. 11a          Fig. 11b    Fig. 11c

Fig. 12a

*Fig-12 b*

*Fig_13a*

*Fig_13b*

FIG. 14a

FIG. 14b

*Fig. 14c*

Fig-14d

*Fig. 14e*

FIG. 15

*12* — Clinical database of patient images (PACs)

Read IS1 *202*

Read IS2 *202*

Co-registration between IS1 and IS2 *204*

Write registration parameter *206*

Aneuryrm segmentation process *208*

*12*

*200*

Fig-16

*32*

$C_R$

R left

*28*

*32*

R right

*36*

$C_{IL}$

*34*

IL left

*34*

IL right

Fig-17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006085155 A